# DEMANDE DE BREVET EUROPEEN

(11) **EP 3 761 028 A1**
(43) Date de publication de la demande: **06.01.2021**
(21) Numéro de dépôt: 20181531.3
(22) Date de dépôt: 23.06.2020
(51) Int. Cl.: G01N 33/24, G01N 1/34, G01N 31/12

(54) **SYSTEME ET PROCEDE POUR LA DETERMINATION DE LA COMPOSITION CHIMIQUE DES COMPOSES CONTENUS DANS UN ECHANTILLON**

(30) Priorité: 04.07.2019 FR 1907457
(71) Demandeur: IFP Energies nouvelles, 92852 Rueil-Malmaison Cedex (FR)
(72) Inventeur: ROMERO-SARMIENTO, Maria-Fernanda, 92852 RUEIL-MALMAISON CEDEX (FR); CADEAU, Pierre, 92852 RUEIL-MALMAISON CEDEX (FR); BEAUMONT, Valerie, 92852 RUEIL-MALMAISON CEDEX (FR); SISSMANN, Olivier, 92852 RUEIL-MALMAISON CEDEX (FR)

(57) **Abrégé**

L'invention concerne un système et un procédé pour séparer en phases liquide et/ou gazeuse des composés d'un échantillon. Le système comprend : un four (D1) pour une chauffe en atmosphère inerte selon une séquence de températures, un premier montage (M1) expérimental, connecté au four (D1) lorsqu'il est en service, comprenant des moyens de circulation de l'effluent issu de la chauffe en atmosphère inerte vers des moyens pour collecter (U) cet effluent, un deuxième montage expérimental (M2), connecté au premier montage expérimental (M1) lorsque le four n'est plus en service, comprenant des moyens pour faire circuler sous vide (PI, P.G., TP) l'effluent collecté par le premier montage vers des moyens (T1, T2) pour séparer en phases liquide et/ou gazeuse l'effluent collecté.

L'invention concerne en outre un système et un procédé pour déterminer une composition chimique de composés chimiques contenus dans un échantillon, au moyen d'un dispositif pour une analyse de la composition chimique des composés des phases liquide et/ou gazeuse séparées.

## Description

### Domaine technique

La présente invention concerne le domaine de l'analyse des composés chimiques d'un échantillon en phase gazeuse, solide et/ou liquide.

En particulier mais de manière non limitative, la présente invention concerne le domaine technique de l'industrie pétrolière, et plus particulièrement le domaine de l'exploration et de l'exploitation d'une formation géologique dans laquelle sont piégés des hydrocarbures. La présente invention peut alors viser à déterminer la composition chimique d'un échantillon de roche de cette formation, notamment pour connaitre précisément la distribution entre les fractions lourdes et légères des composés hydrocarbonés contenus dans cet échantillon. La présente invention peut en outre être complétée par une analyse d'autres types de composés, tels que des composés organo-soufrés.

La présente invention peut également être avantageusement mise en œuvre dans un but environnemental, sur un échantillon d'un sol, pour en connaitre par exemple ses composés polluants.

La présente invention peut toutefois être mise en œuvre sur tout type d'échantillon, tel qu'une huile, un lubrifiant, un échantillon de roches carbonatées, un échantillon de matière organique, un fertilisant organique, une matrice organique et/ou inorganique, dans le but d'en connaitre précisément la composition chimique.

### Technique antérieure

On connaît le dispositif ROCK-EVAL® (IFP Energies nouvelles, France), développé par la demanderesse et décrit notamment dans les brevets FR 2227797 (US 3953171) et FR 2472754 (US 4352673). Le dispositif ROCK-EVAL permet la pyrolyse en atmosphère inerte (non oxydante), selon une séquence de températures prédéfinie, d'un échantillon tel qu'un échantillon de roche sédimentaire. Le four à pyrolyse coopère avec un dispositif de détection et de mesure de la quantité de composés hydrocarbonés de l'échantillon pyrolysé. Le dispositif de détection spécifique comprend, par exemple, un détecteur du type à ionisation de flamme (noté FID). Le détecteur délivre un signal représentatif des quantités de produits hydrocarbonés mesurés. Ce signal peut être transmis à des moyens de calculs, de mémorisation et d'affichage dans lesquels un logiciel spécifique calcule, affiche et mémorise les différents paramètres représentatifs des caractéristiques des hydrocarbures en présence. Le dispositif ROCK-EVAL® comprend en outre un four d'oxydation, pour permettre d'obtenir des informations sur la quantité de carbone organique total (TOC), la quantité de carbone minéral (MinC), ou encore la quantité de SO₂ relatives à l'échantillon considéré.

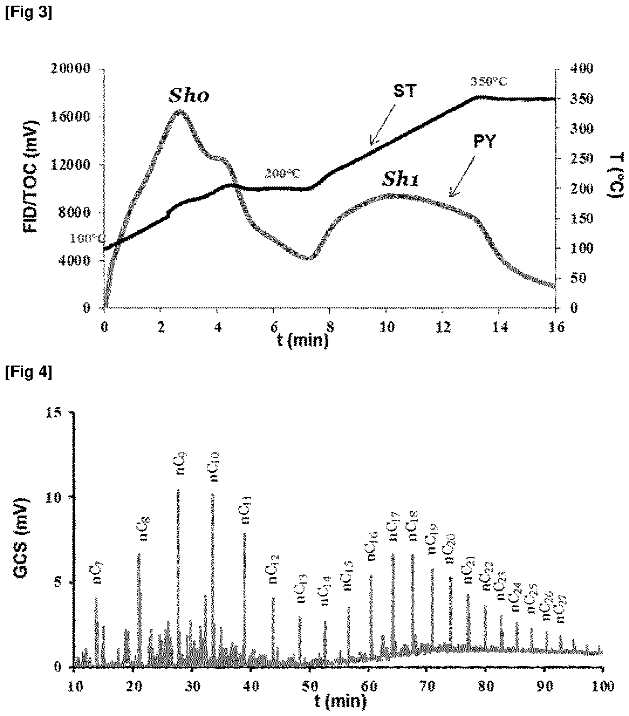

Ainsi, le dispositif ROCK-EVAL® permet en particulier de mesurer la quantité de composés hydrocarbonés libérés tout au long de la pyrolyse. On peut alors établir un pyrogramme, qui est une courbe représentant l'évolution de la quantité de composés hydrocarbonés libérés, rapportée au poids de l'échantillon considéré, en fonction du temps. Un pyrogramme présente généralement plusieurs pics (cf. par exemple les pics Sh0 et Sh1 de la Figure 3) généralement bien différenciés. A partir de la surface d'un de ces pics, on obtient une grandeur représentative de la quantité de composés hydrocarbonés libérés pendant la gamme de température encadrant le pic considéré.

On connaît par ailleurs la méthode "ROCK-EVAL® BULK ROCK", pouvant être mise en œuvre au moyen du dispositif ROCK-EVAL®, et dédiée plus particulièrement aux échantillons conventionnels de roches mères. Cette méthode est notamment décrite dans le document (Behar et al., 2001). La séquence de températures de cette méthode est caractérisée par une température initiale T1 du four de pyrolyse généralement comprise entre 300°C et 350°C, température qui est maintenue pendant une durée prédéterminée de quelques minutes. C'est durant cette phase que sont libérés les hydrocarbures dits « libres » (correspondant en réalité à hydrocarbures de poids moléculaire léger à lourd) initialement contenus dans l'échantillon de roche. Leur quantité est estimée via la mesure de la surface d'un premier pic, noté S₁. Puis, la température de pyrolyse est augmentée progressivement jusqu'à une température T2, généralement de 650°C. Durant cette phase, on assiste à la volatilisation des composés hydrocarbonés très lourds, ainsi qu'au craquage de la matière organique non volatile (kérogène). La quantité de composés hydrocarbonés libérés durant cette phase de craquage thermique est estimée via la mesure de la surface d'un second pic, noté S₂. Elle correspond à la quantité de composés hydrocarbonés qui auraient été générés si la roche avait atteint un stade de maturation suffisant.

On connaît également la méthode "ROCK-EVAL® Reservoir", pouvant également être mise en œuvre au moyen du dispositif ROCK-EVAL®, et dédiée plus particulièrement aux échantillons de roches réservoir et d'huiles. Cette méthode est notamment décrite dans le document EP 0691540 B1 (US 5843787). La séquence de températures de la méthode "ROCK-EVAL® Reservoir" est caractérisée par une température initiale T1 du four de pyrolyse inférieure à 200°C et préférentiellement égale à 180°C. Cette température est maintenue pendant une durée prédéterminée et la quantité de composés hydrocarbonés légers est estimée via la mesure de la surface d'un premier pic, noté S₁ᵣ. Puis, la température du four de pyrolyse est élevée jusqu'à une seconde température T2 d'environ 370°C, phase au cours de laquelle la quantité d'hyd rocarbures plus lourds libérés est estimée via l'estimation de la surface d'un second pic, noté S₂ₐ. La température T2 correspond sensiblement à la fin de la thermovaporisation de certains hydrocarbures et au début du craquage par pyrolyse des composés lourds. Ainsi, la famille de composés hydrocarbonés correspondant aux pics S₁ᵣ et S₂ₐ de la méthode « Réservoir » est quasiment équivalente à la famille de composés hydrocarbonés caractéristiques du pic S₁ de la méthode « Basique », soit des hydrocarbures de poids moléculaire léger à lourd. Puis la température de pyrolyse est à nouveau augmentée jusqu'à une troisième température T3 d'au plus 650°C. La surface d'un troisième pic, noté S_{2b}, représentatif des composés hydrocarbonés lourds, est estimée durant cette troisième phase de chauffe. Ce pic S_{2b} peut être considéré comme un équivalent du pic S₂ de la méthode "ROCK-EVAL® BULK ROCK".

Plus récemment, a été développée la méthode ROCK-EVAL® SHALEPLAY™, décrite notamment dans les brevets 3 021 749 (US 10088465) et les demandes de brevet dont les numéros de dépôt sont FR 17/59.447 (US 16/154.218), pouvant également être mise en œuvre au moyen du dispositif ROCK-EVAL®. Il s'agit de procédés permettant de mieux quantifier les hydrocarbures légers à lourds contenus dans une roche sédimentaire, en particulier dans une roche mère non conventionnelle, et en particulier de quantifier les composés hydrocarbonés, de poids moléculaire légers à lourds, véritablement libres et la quantité de composés hydrocarbonés, de poids moléculaire légers à lourds, retenus dans la matière organique.

Toutefois, de telles analyses réalisées au moyen du dispositif ROCK-EVAL® et de l'une quelconque des méthodes associées ne permet pas de déterminer la composition chimique précise de l'échantillon considéré. Une méthodologie analytique est donc nécessaire pour compléter la détection, la récupération et la quantification de composés chimiques obtenus suite à une analyse réalisée par exemple avec le dispositif ROCK-EVAL®.

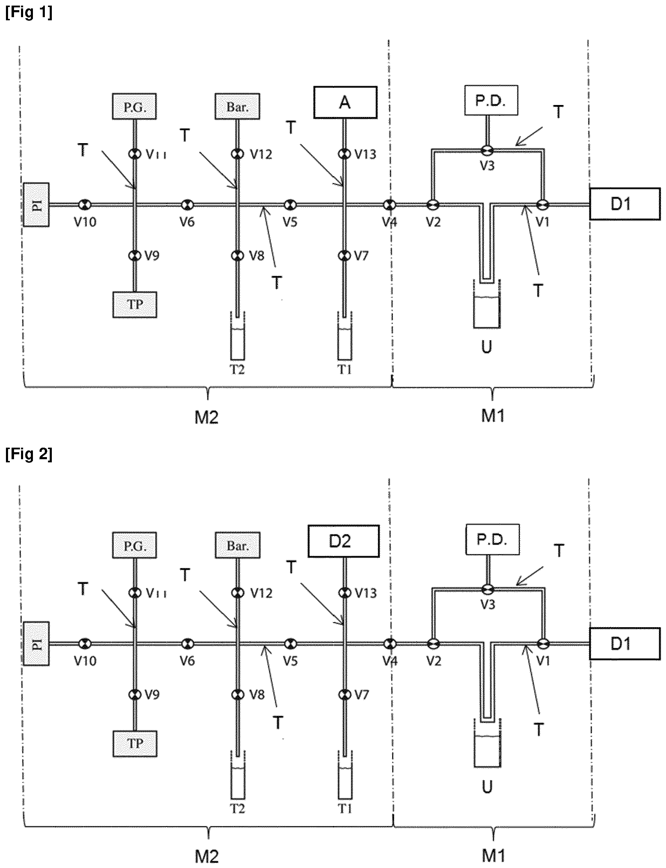

De nombreuses techniques analytiques ont été développées afin de coupler la pyrolyse d'un échantillon à une analyse directe en ligne, comme par exemple avec un chromatographe en phase gazeuse (noté GC, pour Gas Chromatophy) couplé à un détecteur à ionisation de flamme (noté FID, pour Flame lonization Detector) et à un spectromètre de masse à rapport isotopique (noté IRMS, pour Isotope-Ratio Mass Spectrometry). On connait notamment le document :
*Doerner, M., Berner, U., Erdmann, M et Barth, T. (2017). PALEOENVIRONMENTAL CHARACTERIZATION OF THERMALLY MATURE ORGANIC MATTER USING A NEW PYROLYSIS-IRMS METHOD, 28th International Meeting on Organic Geochemistry,* 17 - 22 September 2017, Florence, Italy.
qui combine une unité de pyrolyse, pour chauffer un échantillon de roche sédimentaire selon une séquence de températures donnée, à une analyse en GC/FID-IRMS, en piégeant des fractions d'hydrocarbures libérées par la pyrolyse à l'aide d'un piège refroidi à l'azote liquide. Toutefois, le dispositif décrit dans ce document ne permet pas de faire la séparation, la récupération et la purification de fractions de composés obtenus à la sortie de l'unité de pyrolyse. Notamment, du fait de la séquence de températures utilisée, les hydrocarbures générés entre 100°C et 300°C ne sont pas correcteme nt séparés (pic S1 tronqué en Figure 1 du document Doerner et al, 2017). Par ailleurs, tout au long du reste de la pyrolyse, les différentes fractions d'hydrocarbures qui sont analysés semblent séparées arbitrairement tous les 25°C, sans avoir d'information sur la composition des hydrocarbures qui correspondent à ces différentes fractions. Enfin, l'analyse réalisée dans ce document est destructive, dans le sens où les fractions de composés obtenus à la sortie de l'unité de pyrolyse sont toutes utilisées dans le GC/FID-IRMS, ce qui ne permet pas de réaliser des analyses supplémentaires, ultérieures et/ou déportées.

La présente invention vise à pallier ces inconvénients.

L'invention concerne un système et un procédé pour séparer en phases liquide et/ou gazeuse des composés contenus dans un échantillon, mis en œuvre au moyen d'un premier montage expérimental pour collecter le flux de gaz issu d'une pyrolyse programmée selon une séquence de chauffe prédéfinie et un deuxième montage expérimental sous vide pour séparer et purifier les phases liquide et/ou gazeuse contenues dans ce flux de gaz.

L'invention concerne en outre un système et un procédé pour déterminer la composition chimique des composés contenus dans un échantillon, mis en œuvre au moyen au moins d'un système pour séparer en phases liquide et/ou gazeuse des composés contenus dans un échantillon tel que décrit ci-dessus, pouvant être connecté à tout type d'analyseur de composition chimique placé en aval, tel qu'un chromatographe en phase gazeuse (GC ou GC-FID), un chromatographe en phase gazeuse couplé à un spectromètre de masse (GC-MS), un chromatographe en phase gazeuse couplé à un spectromètre de masse à rapport isotopique (GC-IR-MS), etc.

### Résumé de l'invention

L'invention concerne un système pour séparer en phases liquide et/ou gazeuse des composés contenus dans un échantillon, comprenant au moins
i. un premier dispositif comprenant au moins un four pour réaliser au moins une chauffe en atmosphère inerte selon une séquence de températures prédéfinie, des moyens pour séparer en au moins deux parties un effluent résultant de ladite chauffe en atmosphère inerte, et des moyens de mesure en continu d'au moins d'une quantité de composés hydrocarbonés contenus dans ladite première partie dudit effluent ;
ii. un premier montage expérimental destiné à être connecté audit premier dispositif lorsque ledit premier dispositif est en service, comprenant des moyens pour faire circuler ladite deuxième partie dudit effluent issue dudit premier dispositif vers des moyens pour collecter ladite deuxième partie dudit effluent ;
iii. un deuxième montage expérimental destiné à être connecté audit premier montage expérimental lorsque ledit premier dispositif n'est plus en service, comprenant des moyens pour faire circuler sous vide ladite deuxième partie dudit effluent collectée par ledit premier montage vers des moyens pour séparer en phases liquide et/ou gazeuse ladite deuxième partie dudit effluent collectée.

Selon une mise en œuvre de l'invention, ledit premier dispositif peut comprendre en outre un four d'oxydation pour réaliser une chauffe en atmosphère oxydante.

Selon une mise en œuvre de l'invention, lesdits moyens pour faire circuler ladite deuxième partie dudit effluent issue dudit premier dispositif peuvent comprendre une pompe et un débitmètre.

Selon une mise en œuvre de l'invention, lesdits moyens pour faire circuler ladite deuxième partie dudit effluent collectée par ledit premier montage peuvent comprendre une pompe sous vide.

Selon une mise en œuvre de l'invention, lesdits moyens pour collecter ladite deuxième partie dudit effluent peuvent comprendre un tube en forme de U refroidi par un flux d'azote liquide.

Selon une mise en œuvre de l'invention, lesdits moyens pour réaliser une séparation en phases liquide et/ou gazeuse peuvent comprendre deux tubes refroidis par un flux d'azote liquide disposés en série dans le sens de circulation de ladite deuxième partie dudit effluent dans ledit deuxième montage, ledit tube placé en amont contenant au moins un gel de silice.

Selon une mise en œuvre de l'invention, lesdits premier et/ou deuxième montages expérimentaux peuvent comprendre des moyens de chauffage pour éviter des phénomènes d'adsorption dans lesdits premier et deuxième montages, la gamme de températures desdits moyens de chauffage étant comprise entre environ 100°C et 200°C.

Selon une mise en œuvre de l'invention, ledit deuxième montage expérimental peut comprendre des moyens pour collecter lesdites phases liquides et/ou gazeuses séparées.

Selon une mise en œuvre de l'invention, ledit système peut comprendre des moyens pour isoler respectivement, le premier dispositif et le premier montage expérimental ainsi que le premier montage expérimental et le deuxième montage expérimental, de préférence, les moyens pour isoler étant des vannes, et de manière très préférée des vannes multi-voies.

L'invention concerne en outre un système pour déterminer une composition chimique des composés contenus dans un échantillon, comprenant au moins un système pour séparer en phases liquide et/ou gazeuse des composés contenus dans un échantillon tel que décrit ci-dessus et au moins un deuxième dispositif destiné à être connecté en aval dudit deuxième montage expérimental, ledit deuxième dispositif étant apte à une analyse de la composition chimique des composés contenus dans lesdites phases liquide et/ou gazeuse de ladite deuxième partie dudit effluent séparées.

Selon une mise en œuvre de l'invention, ledit deuxième dispositif peut comprendre un chromatographe en phase gazeuse et/ou un chromatographe en phase gazeuse couplé à un spectromètre de masse et/ou un chromatographe en phase gazeuse couplé à un spectromètre de masse à rapport isotopique.

L'invention concerne en outre un procédé pour séparer en phases liquide et/ou gazeuse des composés contenus dans un échantillon, ledit procédé étant mis en œuvre au moyen d'un système tel que décrit ci-dessus, dans lequel on applique au moins les étapes suivantes :
a. On connecte ledit premier dispositif audit premier montage expérimental et on chauffe ledit échantillon au moins en atmosphère inerte selon une séquence de températures prédéfinie ; de manière continue, pendant ladite chauffe en atmosphère inerte, on sépare en au moins deux parties un effluent résultant de ladite chauffe en atmosphère inerte ; on mesure en continu au moins une quantité de composés hydrocarbonés contenus dans ladite première partie dudit effluent et on collecte en continu ladite deuxième partie dudit effluent au moyen dudit premier montage expérimental ;
b. On déconnecte ledit premier dispositif dudit premier montage expérimental et on connecte ledit premier montage audit deuxième montage et on sépare une phase liquide et/ou une phase gazeuse de ladite deuxième partie dudit effluent au moyen dudit deuxième montage expérimental.
   L'invention concerne en outre un procédé pour déterminer une composition chimique de composés chimiques contenus dans un échantillon, ledit procédé étant mis en œuvre au moyen d'un système pour déterminer une composition chimique des composés contenus dans un échantillon tel que décrit ci-dessus, dans lequel on applique au moins les étapes dudit procédé pour séparer en phases liquide et/ou gazeuse des composés contenus dans un échantillon telles que décrites ci-dessus, et dans lequel on applique en outre au moins l'étape suivante :
c. On transfert lesdites phases liquide et/ou gazeuse séparées au moyen dudit deuxième montage expérimental vers ledit deuxième dispositif et on détermine ladite composition chimique desdits composés contenus respectivement dans ladite phase liquide de ladite deuxième partie dudit effluent et/ou contenus dans ladite phase gazeuse de ladite deuxième partie dudit effluent.

### Liste des figures

La figure 1 présente un exemple de réalisation du système selon le premier aspect de l'invention.
La figure 2 présente un exemple de réalisation du système selon le deuxième aspect de l'invention.
La figure 3 présente un exemple de pyrogramme obtenu selon une séquence de températures prédéfinie.
La figure 4 présente un exemple de chromatogramme en phase gazeuse obtenu à l'issue de la mise en œuvre du procédé selon un troisième aspect de l'invention.

### Description des modes de réalisation

Selon respectivement un premier et un troisième aspects, l'invention concerne un système et un procédé pour séparer en phases liquide et/ou gazeuse des composés contenus dans un échantillon quelconque.

Selon respectivement un deuxième et un quatrième aspects, l'invention concerne un système et un procédé pour déterminer la composition chimique des composés contenus dans un échantillon quelconque, à partir des phases liquides et/ou gazeuses séparées. Selon une première variante de mise en œuvre de l'invention, l'échantillon peut provenir d'une roche sédimentaire d'une formation du sous-sol comprenant des hydrocarbures liquides et/ou gazeux. Dans ce cas, le système et le procédé selon les deuxième et quatrième aspects de l'invention peuvent viser à déterminer la composition chimique au moins des composés hydrocarbonés contenus dans l'échantillon.

Selon une deuxième variante de mise en œuvre de l'invention, l'échantillon provient de diverses matrices organiques et/ou inorganiques comme par exemple des sédiments récents ou bien des sols, des carbonates, des fertilisants, etc.

Selon une troisième variante de mise en œuvre de l'invention, l'échantillon est un échantillon d'un produit pétrolier, tel qu'un lubrifiant ou une huile. Le système et le procédé selon les deuxième et quatrième aspects de l'invention peuvent alors viser à déterminer les composés polluants contenus dans l'échantillon, notamment des polluants de types composés organiques comme les BTEX (Benzène Toluène Éthylbenzène) et/ou les composés organiques volatiles (COV) qui constituent une famille de produits très large trouvés sous forme gazeuse dans l'atmosphère terrestre.

Selon l'invention, l'échantillon peut être liquide et/ou solide. Une roche sédimentaire est par exemple un exemple d'échantillon solide comprenant notamment des composés hydrocarbonés liquides (>C₅) et gazeux (C₁-C₄) si l'échantillon est bien préservé.

Le système selon le premier aspect de l'invention comprend au moins :
a) Un premier dispositif comprenant au moins un four pour réaliser au moins une chauffe en atmosphère inerte selon une séquence de températures prédéfinie, des moyens pour séparer en au moins deux parties un effluent résultant de ladite chauffe en atmosphère inerte, et des moyens de mesure en continu d'au moins d'une quantité de composés hydrocarbonés contenus dans ladite première partie dudit effluent ;
b) un premier montage expérimental destiné à être connecté audit premier dispositif lorsque le premier dispositif est en service (c'est-à-dire en fonctionnement), comprenant des moyens pour faire circuler ladite deuxième partie dudit effluent issue dudit premier dispositif vers des moyens pour collecter ladite deuxième partie dudit effluent ;
c) un deuxième montage expérimental destiné à être connecté audit premier montage expérimental lorsque le premier dispositif n'est plus en service (c'est-à-dire à l'arrêt après son fonctionnement), comprenant des moyens pour faire circuler sous vide ladite deuxième partie dudit effluent collectée par le premier montage vers des moyens pour séparer en phases liquide et/ou gazeuse ladite deuxième partie dudit effluent collectée.

Le système selon le deuxième aspect de l'invention comprend en outre un deuxième dispositif destiné à être connecté en aval du deuxième montage expérimental du système selon le premier aspect de l'invention, le deuxième dispositif étant apte à une analyse de la composition chimique des composés contenus dans lesdites phases liquide et/ou gazeuse de ladite deuxième partie dudit effluent séparées.

Les différents éléments du système selon les premier et deuxième aspects de l'invention sont décrits ci-après.

### Premier dispositif (moyen de chauffe)

Le premier montage expérimental du système selon le premier et/ou le deuxième aspect de l'invention comprend au moins un four pour réaliser au moins une chauffe en atmosphère inerte selon une séquence de températures prédéfinie, des moyens pour séparer en au moins deux parties un effluent résultant de cette chauffe en atmosphère inerte, et des moyens de mesure en continu d'au moins d'une quantité de composés hydrocarbonés contenus dans la première partie de cet effluent. Ainsi le premier dispositif selon l'invention permet au moins une chauffe en atmosphère inerte de l'échantillon étudié et la mesure en continu d'au moins la quantité de composés hydrocarbonés contenus dans cet échantillon. Selon une mise en œuvre de l'invention, le premier dispositif comprend des moyens pour programmer la chauffe du four selon l'invention selon une séquence de températures prédéfinie.

Selon une mise en œuvre de l'invention, la séquence de températures peut être la séquence de température de la méthode dite "ROCK-EVAL® BULK ROCK", telle que notamment décrite dans le document (Behar et al., 2001), ou de la méthode ROCK-EVAL® Reservoir", telle que notamment décrite dans le document EP 0691540 B1 (US 5843787), de la méthode ROCK-EVAL® SHALEPLAY™, telle que décrite notamment dans les brevets FR 3021749 (US 10088465) et les demandes de brevet dont les numéros de dépôt sont FR 17/59.447 (US 16/154218), ou encore toute autre séquence de chauffe.

Selon une mise en œuvre préférée de l'invention, la séquence de chauffe prédéfinie peut être la suivante
a) à partir d'une première valeur de température (T1) comprise entre 50°C et 120°C, on élève la température dudit échantillon selon un premier gradient de température compris entre 1°C/min et 50°C/min, jusqu'à une seconde valeur de température (T2) comprise entre 180°C et 220°C, et on maintient le échantillon à la dite seconde valeur de température (T2) pendant une première durée prédéterminée ;
b) à partir de ladite deuxième valeur de température (T2), on élève la température dudit échantillon selon un deuxième gradient de température compris entre 1°C/min et 50°C/min, jusqu'à une troisième valeur de température (T3) comprise entre 330°C et 370°C, et on maintient le échantillon à ladite troisième valeur de température (T3) pendant une deuxième durée prédéterminée.

Cette version restreinte de la séquence de températures ROCK-EVAL® SHALEPLAY™ permet un gain de temps par rapport à la séquence de températures ROCK-EVAL® SHALEPLAY™ complète, tout en permettant la libération par thermovaporisation des composés hydrocarbonés les plus légers à lourds, qui sont les composés hydrocaronés généralement d'intérêt.

Selon une mise en œuvre de l'invention, les moyens de mesure en continu des composés hydrocarbonés libérés au cours de la pyrolyse peuvent être un détecteur à ionisation de flamme (FID).

Selon une mise en œuvre de l'invention, le premier dispositif du système selon le premier et/ou le deuxième aspect de l'invention peut permettre en outre une chauffe sous atmosphère oxydante. Cette chauffe en atmosphère oxydante peut se faire au moyen du four pour la chauffe en atmosphère inerte selon l'invention, pouvant fonctionner aussi bien en atmosphère inerte qu'en atmosphère oxydante, ou bien au moyen d'un four distinct du four pour la chauffe en atmosphère inerte selon l'invention. Selon cette mise en œuvre de l'invention, le premier dispositif peut comprendre en outre :
- des moyens de mesure en continu de la quantité de SO₂ contenue dans lesdits effluents après oxydation, tels qu'un spectrophotomètre à ultraviolet (UV) ou à infrarouge (IR),
- des moyens de mesure, tels qu'un spectrophotomètre infrarouge (IR), du monoxyde de carbone (CO) et du dioxyde de carbone (CO₂) contenus dans les effluents après oxydation. Préférentiellement, le premier dispositif du système selon le premier et/ou le deuxième aspect de l'invention correspond au dispositif ROCK-EVAL® (IFP Energies nouvelles, France), développée par la demanderesse, et décrit notamment dans le brevet EP 2342557 (US 8796035).

### Premier montage expérimental (moyens de circulation de l'effluent et collecte)

Le premier montage expérimental du système selon le premier et/ou le deuxième aspect de l'invention comprend des moyens pour faire circuler la deuxième partie de l'effluent, issue du premier dispositif selon l'invention, vers des moyens pour collecter cette deuxième partie de l'effluent.

De plus, selon l'invention, ce premier montage expérimental est destiné à être connecté au premier dispositif selon l'invention lorsque le premier dispositif est en service. Selon une mise en œuvre de l'invention, le système peut comprendre des moyens pour isoler le premier dispositif et le premier montage expérimental. De préférence, les moyens pour isoler peuvent être des vannes, et de manière très préférée des vannes multi-voies. Ainsi, ce premier montage expérimental est monté "en ligne" lorsque le premier dispositif est en service.

Selon une mise en œuvre de l'invention, les moyens pour collecter la deuxième partie de l'effluent issue du premier dispositif selon l'invention peuvent comprendre un tube en forme de U ("U-trap" en anglais) refroidi par un flux d'azote liquide.

Selon une mise en œuvre de l'invention, les moyens pour faire circuler la deuxième partie de l'effluent issue du premier dispositif vers les moyens de collecte du premier montage expérimental peuvent comprendre en outre une pompe, un débitmètre et au moins une vanne à 3 voies. Selon une mise en œuvre de l'invention, le gaz vecteur utilisé dans le premier montage expérimental peut être un gaz inerte tel que de l'azote.

Avantageusement, le premier montage expérimental peut comprendre en outre des moyens de chauffage pour éviter des phénomènes d'adsorption dans les différents éléments du premier montage expérimental. Selon une mise en œuvre de l'invention, la gamme de températures des moyens de chauffage peut être comprise entre environ 100°C et 200°C, et vaut préférentiellement 150°C. Selon une mise en œuvre de l'invention, ces moyens de chauffage peuvent être des cordons chauffants.

Selon une mise en œuvre de l'invention, le premier montage expérimental peut comprendre en outre un ou plusieurs tubes en acier inoxydable (par exemple commercialisé par la société Swagelok, Etats-Unis), par exemple d'un diamètre de 1/4 po (environ 6.35mm), dans lesquels circule la deuxième partie de l'effluent issue du premier dispositif selon l'invention.

### Deuxième montage expérimental (moyens de circulation sous vide de l'effluent collecté)

Le deuxième montage expérimental selon l'invention comprend des moyens pour faire circuler sous vide la deuxième partie de l'effluent collectée par le premier montage expérimental vers des moyens pour réaliser une séparation en phases liquide et/ou gazeuse de la deuxième partie de l'effluent collectée. La circulation sous vide permet d'éviter la contamination par l'air et permet ainsi de réaliser un bilan quantitatif précis.

De plus, selon l'invention, ce deuxième montage expérimental est destiné à être connecté au premier montage expérimental lorsque le premier dispositif du système selon le premier et/ou le deuxième aspect de l'invention n'est plus en service. Selon une mise en œuvre de l'invention, le système peut comprendre des moyens pour isoler le premier montage expérimental et le deuxième montage expérimental. De préférence, les moyens pour isoler peuvent être des vannes, et de manière très préférée des vannes multi-voies. Le deuxième montage expérimental est ainsi monté "hors ligne" par rapport au premier dispositif selon l'invention.

Très avantageusement, le système comprend des moyens pour isoler respectivement le premier dispositif et le premier montage expérimental ainsi que le premier montage expérimental et le deuxième montage expérimental, de préférence, les moyens pour isoler étant des vannes, et de manière très préférée des vannes multi-voies.

Selon une mise en œuvre de l'invention, les moyens pour réaliser une séparation en phases liquide et/ou gazeuse de la deuxième partie de l'effluent collectée peuvent comprendre deux tubes refroidis par un flux d'azote liquide disposés en série dans le sens de circulation de la deuxième partie de l'effluent dans le deuxième montage, le tube placé en amont (par rapport au sens de circulation de la deuxième partie de l'effluent collectée circulant dans le deuxième montage expérimental) contenant au moins un gel de silice. Le gel de silice est notamment apte à piéger des gaz, tels que par exemple du méthane.

Selon une mise en œuvre de l'invention, les moyens pour faire circuler sous vide ladite deuxième partie dudit effluent collectée par le premier montage vers les moyens pour séparer en phases liquide et/ou gazeuse peuvent comprendre au moins une pompe sous vide.

Avantageusement, le deuxième montage expérimental peut comprendre en outre des moyens de chauffage pour éviter des phénomènes d'adsorption dans le deuxième montage expérimental. Selon une mise en œuvre de l'invention, la gamme de températures des moyens de chauffage peut être comprise entre environ 100°C et 200°C, et vaut préférentiellement 150°C. Selon une mise en œuvre d e l'invention, les moyens de chauffage du deuxième montage expérimental peuvent être des cordons chauffants.

Selon une mise en œuvre de l'invention, le deuxième montage expérimental peut comprendre en outre un ou plusieurs tubes en acier inoxydable (par exemple les tubes commercialisés par la société Swagelok, Etats-Unis), par exemple d'un diamètre de 1/4 po (environ 6.35mm), dans lesquels circule la deuxième partie de l'effluent après sa collecte par le premier montage expérimental du système selon le premier ou le deuxième aspect de l'invention.

Ainsi, le deuxième montage expérimental du système selon le premier ou le deuxième aspect de l'invention permet une séparation sous vide des phases liquide et/ou gazeuse contenues dans la deuxième partie de l'effluent issue de la mise en œuvre du premier dispositif selon l'invention et collecté par le premier montage expérimental selon l'invention. Avantageusement, le système selon le premier aspect de l'invention peut comprendre en outre des moyens pour collecter les phases liquide et/ou gazeuse ainsi séparées, par exemple au moyen d'ampoules (par exemple en verre) qui peuvent être connectées par exemple via un ou plusieurs tubes en acier inoxydable (par exemple les tubes commercialisés par la société Swagelok, Etats-Unis ; par exemple d'un diamètre de 1/4 po (environ 6.35mm)) et une ou plusieurs vannes multivoies aux moyens pour séparer en phases liquide et/ou gazeuse du deuxième montage expérimental (tels que les tubes refroidis par un flux d'azote liquide azote liquide selon la mise en œuvre de l'invention décrite ci-dessus).

### Deuxième dispositif (moyen d'analyse de la composition chimique)

Le système selon le deuxième aspect de l'invention comprend en outre au moins un deuxième dispositif destiné à être connecté en aval du deuxième montage expérimental selon l'une quelconque des variantes décrites ci-dessus, le deuxième dispositif étant apte à une analyse de la composition chimique des composés contenus dans lesdites phases liquide et/ou gazeuse de ladite deuxième partie dudit effluent séparées.

Selon une mise en œuvre du système selon le deuxième aspect de l'invention, le deuxième dispositif peut correspondre à un chromatographe en phase gazeuse (GC), ce qui permet d'obtenir les différents composés d'un échantillon.

Alternativement ou de manière combinée, le deuxième dispositif du système selon le deuxième aspect de l'invention peut correspondre à un chromatographe en phase gazeuse couplé à un spectromètre de masse (GC-MS), ce qui permet la détection et l'identification des composés en fonction de leur rapport masse sur charge.

Alternativement ou de manière combinée, le deuxième dispositif du système selon le deuxième aspect de l'invention peut correspondre à un chromatographe en phase gazeuse couplé à un spectromètre de masse à rapport isotopique (GC-IRMS), ce qui permet de mesurer l'abondance relative des différents isotopes d'un même élément chimique dans un échantillon donné.

Selon une mise en œuvre du deuxième aspect du système selon l'invention, le deuxième dispositif peut être directement connecté au deuxième montage expérimental par exemple via un ou plusieurs tubes en acier inoxydable (par exemple les tubes commercialisés par la société Swagelok, Etats-Unis ; par exemple d'un diamètre de 1/4 po (environ 6.35mm)) et une ou plusieurs vannes multivoies.

Alternativement, le système selon le deuxième aspect de l'invention comprend en outre des moyens, tel qu'un automate, pour transférer les phases liquide et/ou gazeuse issues du deuxième montage expérimental vers le deuxième dispositif.

Selon un troisième aspect, l'invention concerne un procédé pour séparer les phases liquide et/ou gazeuse des composés contenus dans un échantillon, le procédé pouvant être mis en œuvre au moyen du système pour séparer les phases liquides et/ou gazeuses d'un échantillon selon l'un quelconque des modes de réalisation du premier ou du deuxième aspect de l'invention décrits ci-dessus.

Le procédé pour séparer les phases liquide et/ou gazeuse des composés contenus dans un échantillon selon l'invention comprend au moins les étapes suivantes :
a) Chauffe au moins en atmosphère inerte de l'échantillon et collecte
b) Séparation en phases liquide et gazeuse.

Selon un quatrième aspect, l'invention concerne un procédé pour déterminer la composition chimique des composés contenus dans un échantillon, le procédé pouvant être mis en œuvre au moyen du système pour déterminer la composition chimique des composés contenus dans un échantillon selon l'un quelconque des modes de réalisation du deuxième aspect de l'invention décrits ci-dessus. Le procédé pour la détermination de la composition chimique des composés contenus dans un échantillon (quatrième aspect) comprend la mise en œuvre au moins des étapes a) et b) du procédé pour séparer les phases liquides et/ou gazeuses des composés d'un échantillon selon l'une quelconque des variantes qui seront décrites ci-dessous, et au moins une étape c) supplémentaire consistant en l'analyse des composés chimiques des phases liquide et/ou gazeuse issues de l'étape b).

Les étapes des procédés selon les troisième et/ou un quatrième aspects de l'invention sont décrites ci-après.

### a) Chauffe au moins en atmosphère inerte de l'échantillon et collecte

Au cours de cette étape, on connecte le premier dispositif du système selon le premier et/ou le deuxième aspect de l'invention au premier montage expérimental et on chauffe l'échantillon au moins en atmosphère inerte selon une séquence de températures prédéfinie. Puis, de manière continue pendant cette chauffe en atmosphère inerte, on sépare en au moins deux parties un effluent résultant de cette chauffe en atmosphère inerte. On mesure alors en continu au moins une quantité de composés hydrocarbonés contenus dans la première partie de l'effluent et on collecte en continu la deuxième partie de l'effluent au moyen du premier montage expérimental.

Selon une mise en œuvre de l'invention, la séquence de températures prédéfinie peut être la séquence de température de la méthode dite "ROCK-EVAL® BULK ROCK", telle que notamment décrite dans le document (Behar et al., 2001), ou de la méthode ROCK-EVAL® Reservoir", telle que notamment décrite dans le document EP 0691540 B1 (US 5843787), ou de la méthode ROCK-EVAL® SHALEPLAY™, telle que décrite notamment dans les brevets FR 3021749 (US 10088465) et dans les demandes de brevet dont les numéros de dépôt sont FR 17/59.447 (US 16/154218), ou encore toute autre séquence de chauffe.

Selon une mise en œuvre préférée selon laquelle le premier dispositif du système selon l'invention est un dispositif ROCK-EVAL®, le flux de gaz de composés hydrocarbonés libérés par la séquence de chauffe en atmosphère inerte est divisé en deux parties, une première partie qui est envoyée vers un détecteur à ionisation de flamme (FID) du dispositif ROCK-EVAL®, et une deuxième partie qui est envoyée vers le premier montage expérimental du système selon l'invention, pour être collectée par exemple dans un tube en forme de U refroidi par de l'azote liquide.

Selon une mise en œuvre de l'invention, cette étape peut avoir une durée comprise entre 10 minutes et 40 minutes, et vaut préférentiellement 25 minutes. De telles durées permettent de s'assurer que la majorité des composés chimiques thermovaporisables et/ou pyrolysables contenus dans l'échantillon considéré ait été libérée puis collectée.

Avantageusement, pendant cette phase de collecte au moyen du premier montage expérimental, on maintient au moins une partie des éléments du premier montage expérimental (plus précisément, au moins à l'exclusion de l'élément dédié à la collecte de l'effluent, tel que le tube en forme de U dans le mode préféré du premier montage expérimental) à une température comprise entre 100 et 200°C, et valant préférentiellement 150 °C, au moyen par exemple de cordons chauffants. Ceci permet d'éviter toute adsorption des composés libérés par le premier dispositif sur les parois des éléments du premier montage expérimental (hors celui dédié à la collecte de l'effluent, tel que le tube en forme de U dans le mode préféré du premier montage expérimental).

### b) Séparation en phases liquide et/ou gazeuse

Au cours de cette étape, on déconnecte le premier dispositif du premier montage expérimental du système selon le premier et/ou le deuxième aspect de l'invention et on connecte le premier montage expérimental au deuxième montage expérimental du système selon les premier et/ou le deuxième aspects de l'invention.

Avantageusement, ceci peut être réalisé au moyen de moyens d'isolement des premier et/ou deuxième montages expérimentaux, ces moyens d'isolement pouvant être des vannes, et de manière très préférée de vannes multi-voies.

Puis on sépare une phase liquide et/ou une phase gazeuse de la deuxième partie de l'effluent au moyen du deuxième montage expérimental du système selon le premier et/ou le deuxième aspect de l'invention, qui comprend au moins des moyens pour faire circuler sous vide la deuxième partie de l'effluent collectée par le premier montage vers des moyens pour séparer en phases liquide et/ou gazeuse un effluent.

Selon une mise en œuvre préférée du système selon l'invention, le deuxième montage expérimental du système selon le premier et/ou le deuxième aspect de l'invention peut comprendre deux tubes refroidis par un flux d'azote liquide (le flux d'azote liquide est appliqué à l'extérieur des deux tubes). Selon ce mode de mise en œuvre, les tubes sont disposés en série dans le sens de circulation du flux engendré par les moyens de circulation du deuxième montage expérimental, le tube placé en amont (par rapport au sens de circulation du flux engendré par les moyens de circulation du deuxième montage expérimental) contenant au moins un gel de silice. Le gel de silice est notamment apte à piéger des gaz, tels que par exemple du méthane. Ainsi, on recueille d'abord la phase gazeuse de la deuxième partie de l'effluent dans le premier tube du deuxième montage expérimental, situé en amont par rapport au sens de circulation, puis on recueille dans le deuxième tube, situé en aval par rapport au sens de circulation, la phase liquide de la deuxième partie de l'effluent.

Selon une mise en œuvre de l'invention, cette étape peut avoir une durée comprise entre 5 et 30 minutes et vaut préférentiellement 15 minutes, de manière à permettre une stabilisation dans chacun des deux tubes du deuxième montage expérimental.

Avantageusement, pendant la circulation sous vide de la deuxième partie de l'effluent dans le deuxième montage expérimental, on peut maintenir au moins une partie des éléments du deuxième montage expérimental (plus précisément, au moins à l'exclusion d'éléments dédiés à la collecte des phases gazeuse et/ou liquide une fois séparées) à une température comprise entre 100 et 200°C, et valant préférentiel lement 150 °C, au moyen par exemple de cordons chauffants. Ceci permet d'éviter toute adsorption des composés présents dans la deuxième partie de l'effluent sur les parois des différents éléments du deuxième montage expérimental (hors ceux dédiés à la collecte des phases gazeuse et liquide une fois séparées). Selon la mise en œuvre préférée du système selon le premier et/ou le deuxième aspect de l'invention selon laquelle le deuxième montage expérimental comprend deux tubes refroidis par un flux d'azote liquide, ces tubes peuvent toutefois ne pas être maintenus à la température comprise entre 100 et 200°C pendant cet te phase de circulation sous vide de cette deuxième partie de l'effluent.

Avantageusement, lorsque les phases liquide et/ou gazeuse du deuxième effluent ont été séparées (c'est-à-dire au bout d'environ 15 minutes), on peut arrêter la circulation sous vide dans le deuxième montage expérimental et on collecte les phases liquide et/ou gazeuse ainsi séparées.

Selon la mise en œuvre préférée du système selon l'un des aspects de l'invention selon laquelle le deuxième montage expérimental comprend deux tubes refroidis par un flux d'azote liquide, le contenu des deux tubes peut être transféré à des moyens pour collecter les phases liquide et/ou gazeuse ainsi séparées (comme par exemple des ampoules, notamment en verre), via un ou plusieurs tubes en acier inoxydable (par exemple les tubes commercialisés par la société Swagelok, Etats-Unis ; par exemple d'un diamètre de 1/4 po (environ 6.35mm)) et une ou plusieurs vannes multivoies. Avantageusement, cette phase de collecte des phases liquide et/ou gazeuse peut être accélérée en chauffant ces deux tubes à une température comprise entre 100 et 200°C, valant préférentiellement 150 °C, au moyen par exemple de cordons chauffants.

### c) Analyse des composés chimiques des phases liquide et/ou gazeuse

Cette étape est mise en œuvre dans le cadre du procédé pour déterminer la composition chimique des composés contenus dans un échantillon (quatrième aspect de l'invention).

Au cours de cette étape, on transfert les phases liquide et/ou gazeuse séparées au moyen du deuxième montage expérimental vers le deuxième dispositif du système pour déterminer la composition chimique des composés contenus dans un échantillon (deuxième aspect de l'invention) et on détermine la composition chimique des composés contenus respectivement dans la phase liquide de la deuxième partie de l'effluent et/ou contenus dans la phase gazeuse de la deuxième partie de l'effluent.

Avantageusement, lorsque les phases liquide et/ou gazeuse du deuxième effluent ont été séparées (c'est-à-dire au bout d'environ 15 minutes), on peut arrêter la circulation sous vide dans le deuxième montage expérimental et on peut transférer les phases liquide et gazeuse recueillies vers le deuxième dispositif du système selon l'invention.

Selon la mise en œuvre préférée du système selon l'invention selon laquelle le deuxième montage expérimental comprend deux tubes refroidis par un flux d'azote liquide, le contenu des deux tubes est transféré vers le deuxième dispositif du système selon l'invention par exemple en chauffant les deux tubes à une température comprise entre 100 et 200°C, valant préférentiellement 150 °C, au moyen par exemple de cordons chauffants.

Les composés hydrocarbonés libérés par la chauffe au moins en atmosphère inerte de l'échantillon au moyen du premier dispositif, piégés par le premier montage expérimental, puis séparés en phase liquide et/ou gazeuse par le deuxième montage expérimental, peuvent ensuite être analysés par un chromatographe en phase gazeuse (GC ou GC-FID), un chromatographe en phase gazeuse couplé à un spectromètre de masse (GC-MS), un chromatographe en phase gazeuse couplé à un spectromètre de masse à rapport isotopique (GC-IR-MS), ou tout autre analyseur.

La figure 1 présente un exemple de réalisation non limitatif du système selon le premier aspect de l'invention, qui comprend :
i. un premier dispositif D1, qui est de manière préférée un dispositif ROCK-EVAL® tel que décrit ci-dessus ;
ii. un premier montage expérimental M1, dit "en ligne". Selon cette mise en œuvre de l'invention, le premier montage expérimental est composée par les éléments suivants : un tube T « Swagelok » en acier inoxydable avec un piège U en forme de U U, des tubes T « Swagelok » en acier inoxydable avec 3 robinets sphérique à 3 voies V1, V2 et V3, une pompe associée à un débitmètre P.D., et des cordons chauffants (non représentés). La vanne V1 permet une connexion au premier dispositif D1.
iii Un deuxième montage expérimental M2, dit "hors ligne. Ce deuxième montage est composé par des tubes T « Swagelok » en acier inoxydable (avec unions croix et tés, raccords et connexions en acier inoxydable « Swagelok »), des vannes V4 à V13 de type VCR avec joint étanches « Swagelok », deux pièges à tubes en acier inoxydable (T1 et T2 contenant du gel de silice), deux pompes - une primaire PI et une secondaire turbo TP - une jauge à vide P.G. de type « Pfeiffer-1 », un manomètre Bar. de type« Baratron-1 » et des cordons chauffants (non représentés). Ce deuxième montage hors ligne, sous vide, permet de purifier et séparer, si nécessaire, les composés gazeux des composés liquides. Cette mise en œuvre du système selon le premier aspect de l'invention comprend aussi une ampoule A pour recueillir par exemple la phase gazeuse et/ou la phase liquide ainsi séparées.

La figure 2 présente un exemple de réalisation non limitatif du système selon le deuxième aspect de l'invention, qui comprend un dispositif D1 et des montages expérimentaux M1 et M2 tels que décrits ci-dessus pour le mode de réalisation du système selon le premier aspect de l'invention, à l'exception du fait que l'ampoule de collecte A est remplacée par un dispositif D2 apte à une analyse de la composition chimique des composés contenus dans lesdites phases liquide et/ou gazeuse, comme par exemple un chromatographe en phase gazeuse.

Un exemple non limitatif de mise en œuvre du procédé selon le troisième aspect de l'invention est décrit ci-après, cette mise en œuvre étant réalisée au moyen du système selon le premier aspect de l'invention dont une réalisation est montrée en Figure 1 (notamment les signes de référence cités sont ceux de la Figure 1):
i. On réalise une pyrolyse en atmosphère inerte selon la séquence de chauffe de la méthode ROCK-EVAL® SHALEPLAY™ restreinte, telle que dans le mode de réalisation préféré décrit ci-dessus, sur l'échantillon à analyser au moyen du dispositif D1 de type ROCK-EVAL® tel que décrit ci-dessus. Au cours de cette analyse, le flux de gaz de composés hydrocarbonés est divisé en deux : une partie est envoyée vers un détecteur à ionisation de flamme du dispositif ROCK-EVAL® et l'autre partie est envoyée vers le premier montage expérimental M1 couplé au ROCK-EVAL® D1. En parallèle à la chauffe en atmosphère inerte réalisée au moyen du dispositif ROCK-EVAL® D1, on applique un flux d'azote continu dans le premier montage expérimental M1, qui est couplé au dispositif ROCK-EVAL® D1 pendant la phase de fonctionnement du dispositif ROCK-EVAL® D1. Ce flux d'azote continu est imposé par la pompe P.D. associée à un débitmètre. Les composés (ex. thermo-vaporisés et/ou pyrolysés) envoyés dans le premier montage expérimental M1 traversent le piège. Afin de piéger tous les composés hydrocarbonés, ce piège U est refroidi grâce à la température de l'azote liquide, sans piéger le gaz vecteur circulant dans le premier montage expérimental M1. Le pompage associé au dispositif ROCK-EVAL® peut rester opérationnel, pour terminer le cycle de pyrolyse et/ou d'oxydation souhaités, et compléter ainsi l'analyse de l'échantillon donné.
ii. Lorsque le programme de températures du dispositif ROCK-EVAL® D1 est terminé, le piège U est isolé du dispositif ROCK-EVAL® en utilisant la vanne V1, puis le piège U est connecté au deuxième montage expérimental M2 via les vannes V2 et V4. Lorsque le piège U est connecté au deuxième montage expérimental M2, la partie du premier montage expérimental M1, connectée au deuxième montage expérimental M2, est chauffée à environ 150°C +/- 50°C. Les composés préalablement piégés s ont libérés et transférés dans le circuit du deuxième montage expérimental M2, et notamment dans les deux tubes T1 et T2 refroidis à la température de l'azote liquide. Le tube T1 contient du gel de silice pour piéger le méthane. Après un temps de stabilisation (environ 15 minutes), les composés piégés dans ces tubes T1 et T2 sont isolés du reste du deuxième montage expérimental M2 en utilisant les vannes V7 et V8. Le reste de la ligne est placée sous pompage via les pompes PI et TP. L'ensemble de la ligne, à l'exception des deux tubes pièges T1 et T2 à froid, peut être chauffée par des cordons chauffants à environ 150°C +/- 50°C afin d'éviter toute perte des composés par effets d'adsorption sur les tubes en acier inoxydable.
iii. Finalement, pour récupérer les composés hydrocarbonés, le pompage est interrompu et les tubes de piégeage T1 et T2 sont reconnectés à la ligne, en utilisant les vannes V7 et/ou V8, pour récupérer seulement le méthane, ou tous les hydrocarbures. Les pièges T1 et T2 sont ensuite chauffés pour accélérer la libération d'hydrocarbures au moyen de cordons chauffants. Après un temps de stabilisation (environ 15 minutes), l'ampoule A pour la récupération d'une des phases, liquide ou gazeuse, est isolée via la vanne V13 et retirée, pour poursuivre éventuellement les analyses, par exemple par chromatographie en phase gazeuse ou toute autre analyse.

Ainsi, les procédés et systèmes selon l'invention permettent de piéger l'intégralité des effluents issus d'une pyrolyse fonctionnant sous flux d'azote, puis de purifier/concentrer ces effluents en pompant le gaz vecteur et autres potentiels contaminants avant d'introduire les effluents d'intérêt dans le deuxième montage expérimental sous vide.

Par ailleurs, les procédé et système pour séparer en phases liquides et/ou gazeuses les composés d'un échantillon permettent une analyse non destructive, en conservant les phases liquides et/ou gazeuses ainsi séparées (par exemple dans des ampoules en verre), en vue d'une ou plusieurs analyses supplémentaires, ultérieures et/ou déportées. En effet, à l'issue du procédé selon le troisième aspect de l'invention, on peut utiliser la totalité des phases liquide et/ou gazeuse ainsi collectées pour une unique analyse, ou bien on peut diviser les phases liquides et/ou gazeuses ainsi collectés pour réaliser différents types d'analyses (comme par exemple une chromatographe en phase gazeuse, un chromatographe en phase gazeuse couplé à un spectromètre de masse, un chromatographe en phase gazeuse couplé à un spectromètre de masse à rapport isotopique) permettant par exemple des conclusions complémentaires. Les procédé et système pour séparer en phases liquides et/ou gazeuses les composés d'un échantillon selon l'invention sont en outre particulièrement avantageux dans le cas d'analyses de composition chimique qui ne pourraient être réalisées directement à la suite de leur collecte, du fait d'un long temps d'analyse et/ou d'un matériel présent dans des laboratoires externes.

De manière générale, les systèmes selon les premier et deuxième aspects de l'invention peuvent être mis en œuvre au moyen de tout type d'unité de pyrolyse. De manière générale, le système selon le deuxième aspect de l'invention peut être mis en œuvre au moyen de tout type de dispositif apte à une analyse de la composition chimique des composés contenus dans lesdites phases liquide et/ou gazeuse. Ainsi, les systèmes selon l'invention offrent une grande flexibilité.

### Exemples

Les avantages des procédés et systèmes selon l'invention sont présentés ci-après.

Le procédé selon le troisième aspect de l'invention a été appliqué à un échantillon d'huile, au moyen du mode de réalisation du système selon le premier aspect de l'invention décrit ci-dessus à la figure 1. Puis, on a recueilli le contenu de la phase liquide dans l'ampoule A et on l'a transféré pour une analyse par un chromatographe en phase gazeuse.

La figure 3 présente un pyrogramme PY représentatif du signal FID/TOC (signal mesuré par un FID, normalisé par le taux de carbone organique) issu d'une analyse réalisée au moyen du dispositif ROCK-EVAL® selon la séquence de chauffe ST préférée, en l'espèce la séquence ROCK-EVAL® SHALEPLAY™ restreinte, telle que décrite dans le mode de réalisation préféré ci-dessus et représentée également en Figure 3.

On peut observer sur cette figure :
- un premier pic, noté Sh0, correspondant aux composés hydrocarbonés libérés pendant la première rampe de chauffe entre les températures 100 et 200°C et le premier plateau (à 200°C) de la séquence de chauffe préférée ST.
- un deuxième pic, noté Sh1, correspondant aux composés hydrocarbonés libérés pendant la deuxième rampe de chauffe (entre les températures 200 et 350°C) et le deuxième plateau (à 350°C) de la séquence de chauffe préférée ST.

De manière générale, un tel pyrogramme représente la quantité de composés hydrocarbonés libérés au cours du temps par thermovaporisation, selon la séquence de chauffe choisie. En particulier, la surface du pic Sh0 (donnée par exemple en milligramme de composés hydrocarbonés par gramme de roche) est représentative de la quantité des hydrocarbures thermovaporisables les plus légers (dont le nombre d'atomes de carbone est inférieur à 20) contenus dans l'échantillon considéré. La surface du pic Sh1 (donnée par exemple en milligramme de composés hydrocarbonés par gramme de roche) est représentative des hydrocarbures thermovaporisables lourds (dont le nombre d'atomes de carbone est compris entre 20 et 30).

La figure 4 présente un chromatogramme en phase gazeuse obtenu après analyse du contenu de l'ampoule A dans lequel on a transféré uniquement la phase liquide présente dans le tube T2. Un chromatogramme en phase gazeuse est obtenu au moyen d'un chromatographe en phase gazeuse (GC), qui permet de détecter et séparer des molécules d'un mélange. Ces informations sont donc complémentaires du pyrogramme PY obtenu à l'issue de l'étape de chauffe au moins en atmosphère inerte. En particulier, l'analyse du chromatogramme permet de conclure sur les types de composés hydrocarbonés contenus dans la phase liquide. Notamment, l'analyse conjointe des Figures 3 et 4 permet de conclure que, parmi les composés les plus légers contribuant au pic Sh0 du pyrogramme, il n'y a que très peu de composés hydrocarbonés ayant un nombre d'atomes de carbone inférieur à 7 (premier pic significatif du chromatogramme correspondant aux composés hydrocarbonés ayant un nombre d'atomes de carbone égal à 7, noté nC7 en Figure 4).

Ainsi, la mise en œuvre du procédé selon le troisième aspect de l'invention, complétée avec une analyse de la composition chimique des composés contenus les phases liquides et/ou gazeuses séparées, permet une caractérisation quantitative et qualitative des composés organiques libérés par pyrolyse. De plus, lorsque le système selon l'invention comprend des moyens pour collecter les phases liquide et/ou gazeuse ainsi séparées (tels qu'une ampoule), on peut conserver les phases liquides et/ou gazeuses ainsi séparées pour une ou plusieurs analyses ultérieures, par exemple via un chromatographe en phase gazeuse, un chromatographe en phase gazeuse couplé à un spectromètre de masse et/ou un chromatographe en phase gazeuse couplé à un spectromètre de masse à rapport isotopique, ces dispositifs pouvant qui plus est être déportés dans l'espace.

## Revendications

1. Système pour séparer en phases liquide et/ou gazeuse des composés contenus dans un échantillon, comprenant au moins :
i) un premier dispositif (D1) comprenant au moins un four pour réaliser au moins une chauffe en atmosphère inerte selon une séquence de températures prédéfinie, des moyens pour séparer en au moins deux parties un effluent résultant de ladite chauffe en atmosphère inerte, et des moyens de mesure en continu d'au moins d'une quantité de composés hydrocarbonés contenus dans ladite première partie dudit effluent ;
ii) un premier montage expérimental (M1) destiné à être connecté audit premier dispositif (D1) lorsque ledit premier dispositif (D1) est en service, comprenant des moyens pour faire circuler (P.D.) ladite deuxième partie dudit effluent issue dudit premier dispositif (D1) vers des moyens pour collecter (U) ladite deuxième partie dudit effluent ;
iii) un deuxième montage expérimental (M2) destiné à être connecté audit premier montage expérimental (M1) lorsque ledit premier dispositif (D1) n'est plus en service, comprenant des moyens pour faire circuler sous vide (PI, TP, P.G.) ladite deuxième partie dudit effluent collectée par ledit premier montage (M1) vers des moyens pour séparer en phases liquide et/ou gazeuse (T1, T2) ladite deuxième partie dudit effluent collectée.

2. Système pour séparer en phases liquide et/ou gazeuse des composés contenus dans un échantillon selon la revendication 1, dans lequel ledit premier dispositif (D1) comprend en outre un four d'oxydation pour réaliser une chauffe en atmosphère oxydante.

3. Système pour séparer en phases liquide et/ou gazeuse des composés contenus dans un échantillon selon l'une des revendications précédentes, dans lequel, dans ledit premier montage (M1), lesdits moyens pour faire circuler ladite deuxième partie dudit effluent issue dudit premier dispositif comprennent une pompe et un débitmètre (P.D.).

4. Système pour séparer en phases liquide et/ou gazeuse des composés contenus dans un échantillon selon l'une des revendications précédentes, dans lequel, dans ledit deuxième montage (M2), lesdits moyens pour faire circuler ladite deuxième partie dudit effluent collectée par ledit premier montage comprennent une pompe sous vide (PI, TP).

5. Système pour séparer en phases liquide et/ou gazeuse des composés contenus dans un échantillon selon l'une des revendications précédentes, dans lequel, dans ledit premier montage (M1), lesdits moyens pour collecter ladite deuxième partie dudit effluent comprennent un tube (U) en forme de U refroidi par un flux d'azote liquide.

6. Système pour séparer en phases liquide et/ou gazeuse des composés contenus dans un échantillon selon l'une des revendications précédentes, dans lequel, dans ledit deuxième montage (M2), lesdits moyens pour réaliser une séparation en phases liquide et/ou gazeuse comprennent deux tubes (T1, T2) refroidis par un flux d'azote liquide disposés en série dans le sens de circulation de ladite deuxième partie dudit effluent dans ledit deuxième montage (M2), ledit tube placé en amont (T1) contenant au moins un gel de silice.

7. Système pour séparer en phases liquide et/ou gazeuse des composés contenus dans un échantillon selon l'une des revendications précédentes, dans lequel, lesdits premier (M1) et/ou deuxième (M2) montages expérimentaux comprennent des moyens de chauffage pour éviter des phénomènes d'adsorption dans lesdits premier et deuxième montages, la gamme de températures desdits moyens de chauffage étant comprise entre environ 100°C et 200°C.

8. Système pour séparer en phases liquide et/ou gazeuse des composés contenus dans un échantillon selon l'une des revendications précédentes, dans lequel, ledit deuxième montage expérimental (M2) comprend des moyens pour collecter (A) lesdites phases liquides et/ou gazeuses séparées.

9. Système pour séparer en phases liquide et/ou gazeuse des composés contenus dans un échantillon selon l'une des revendications précédentes, dans lequel ledit système comprend des moyens (V1 à V13) pour isoler respectivement, le premier dispositif (D1) et le premier montage expérimental (M1) ainsi que le premier montage expérimental (M1) et le deuxième montage expérimental (M2), de préférence, les moyens pour isoler étant des vannes, et de manière très préférée des vannes multi-voies (V1 à V13).

10. Système pour déterminer une composition chimique des composés contenus dans un échantillon, comprenant au moins un système pour séparer en phases liquide et/ou gazeuse des composés contenus dans un échantillon selon l'une quelconque des revendications 1 à 9 et au moins un deuxième dispositif (D2) destiné à être connecté en aval dudit deuxième montage expérimental (M2), ledit deuxième dispositif (D2) étant apte à une analyse de la composition chimique des composés contenus dans lesdites phases liquide et/ou gazeuse de ladite deuxième partie dudit effluent séparées.

11. Système pour la détermination de la composition chimique des composés contenus dans un échantillon selon la revendication 10, dans lequel ledit deuxième dispositif (D2) comprend un chromatographe en phase gazeuse et/ou un chromatographe en phase gazeuse couplé à un spectromètre de masse et/ou un chromatographe en phase gazeuse couplé à un spectromètre de masse à rapport isotopique.

12. Procédé pour séparer en phases liquide et/ou gazeuse des composés contenus dans un échantillon, ledit procédé étant mis en œuvre au moyen du système selon l'une quelconque des revendications 1 à 9, dans lequel on applique au moins les étapes suivantes :
a) On connecte ledit premier dispositif (D1) audit premier montage expérimental (M1) et on chauffe ledit échantillon au moins en atmosphère inerte selon une séquence de températures prédéfinie ; de manière continue, pendant ladite chauffe en atmosphère inerte, on sépare en au moins deux parties un effluent résultant de ladite chauffe en atmosphère inerte ; on mesure en continu au moins une quantité de composés hydrocarbonés contenus dans ladite première partie dudit effluent et on collecte en continu ladite deuxième partie dudit effluent au moyen dudit premier montage expérimental (M1) ;
b) On déconnecte ledit premier dispositif (D1) dudit premier montage expérimental (M1) et on connecte ledit premier montage (M1) audit deuxième montage (M2) et on sépare une phase liquide et/ou une phase gazeuse de ladite deuxième partie dudit effluent au moyen dudit deuxième montage expérimental (M2).

13. Procédé pour déterminer une composition chimique de composés chimiques contenus dans un échantillon, ledit procédé étant mis en œuvre au moyen du système selon l'une quelconque des revendications 10 à 11, dans lequel on applique au moins les étapes du procédé pour séparer en phases liquide et/ou gazeuse des composés contenus dans un échantillon selon la revendication 12, et dans lequel on applique en outre au moins l'étape suivante :
c) On transfert lesdites phases liquide et/ou gazeuse séparées au moyen dudit deuxième montage expérimental (M2) vers ledit deuxième dispositif (D2) et on détermine ladite composition chimique desdits composés contenus respectivement dans ladite phase liquide de ladite deuxième partie dudit effluent et/ou contenus dans ladite phase gazeuse de ladite deuxième partie dudit effluent.
